# EUROPÄISCHE PATENTSCHRIFT

(11) **EP 3 224 244 B1**
(45) Veröffentlichungstag und Bekanntmachung des Hinweises auf die Patenterteilung: **30.01.2019**
(21) Anmeldenummer: 15801785.5
(22) Anmeldetag: 26.11.2015
(51) Int. Cl.: C07C 409/34, C07C 407/00

(54) **VERFAHREN ZUR HERSTELLUNG VON PULVERFÖRMIGEM LAUROYLPEROXID**
METHOD FOR PRODUCING POWDERY LAUROYL PEROXIDE
PROCÉDÉ DE PRODUCTION DE PEROXYDE DE LAURYLE EN POUDRE

(30) Priorität: 28.11.2014 EP 14195444
(43) Veröffentlichungstag der Anmeldung: 04.10.2017
(73) Patentinhaber: UNITED INITIATORS GMBH, 82049 Pullach (DE)
(72) Erfinder: HERMANN, Dominik, 85221 Dachau (DE); NAGL, Iris, 81377 München (DE); WOLF, Hanno, 85521 Ottobrunn (DE)
(74) Vertreter: Weickmann & Weickmann PartmbB
(86) Internationale Anmeldenummer: PCT/EP2015/077789
(87) Internationale Veröffentlichungsnummer: WO 2016/083514

(56) Entgegenhaltungen:
- DD-A- 38 072
- DE-A1- 1 768 199
- GB-A- 950 978
- GB-A- 1 135 372
- GB-A- 1 198 424
- GB-A- 2 054 583
- DATABASE WPI Week 197450 Thomson Scientific, London, GB; AN 1974-86120V XP002753910, & JP S49 36593 A (WAKO PURE CHEM INDS LTD) 4. April 1974 (1974-04-04)

## Beschreibung

Die vorliegende Anmeldung betrifft ein Verfahren zur Herstellung von pulverförmigem Lauroylperoxid, welches dadurch gekennzeichnet ist, dass ein Reaktionsgemisch, das Wasser, Laurinsäurechlorid, Wasserstoffperoxid, anorganische Base und ein Alkan umfasst, unter speziellen Bedingungen umgesetzt wird.

Lauroylperoxid (alternative Bezeichnungen: Didodecanoylperoxid, Dilauroylperoxid) ist ein für Polymerisationsreaktionen verwendeter Initiator, der breite Anwendung findet. Es kann beispielsweise als farbloses, grobes Pulver vorliegen, welches in Wasser unlöslich, jedoch in Ölen und vielen organischen Lösungsmitteln löslich ist. Sein Schmelzpunkt liegt im Bereich von ungefähr 48°C bis 54°C. Generell sind organische Peroxide thermisch labile Verbindungen, die exotherm unter Spaltung der peroxidischen Sauerstoff-Sauerstoff-Bindung zerfallen. So ist Lauroylperoxid stark reaktionsfähig. Im Temperaturbereich des Schmelzpunkts liegt auch die kritische Temperatur dieser Verbindung, bei der eine sich selbst beschleunigende Zersetzung beginnt (engl. *self accelerating decomposition temperature,* SADT).

Lauroylperoxid kann aus Laurinsäurechlorid und Wasserstoffperoxid in Gegenwart von wässrigen Lösungen anorganischer Basen hergestellt werden. Synthese, Aufarbeitung und Formulierung von Lauroylperoxid sind in zahlreichen Publikationen beschrieben.

DD 38072 beschreibt die kontinuierliche Herstellung von Lauroylperoxid aus Laurinsäurechlorid und Wasserstoffperoxid in einer Rührkesselkaskade bei 60°C. Zur Aufarbeitung lässt man das geschmolzene Lauroylperoxid in kaltes Wasser fließen und zentrifugiert das erstarrte Produkt ab.

Das ebenfalls kontinuierliche Verfahren nach DE 29 28 020 A1 arbeitet in der Synthese bei 0 bis 20°C. Zur Aufarbeitung muss das Lauroylperoxid über seinen Schmelzpunkt hinaus erwärmt werden. Die Schmelze kann auf einem Separator abgetrennt werden.

DE 1 192 181 stellt ein Herstellungsverfahren im Lösungsmittel Heptan vor, das bei einer Synthesetemperatur von 40 bis 65°C arbeitet. Nach der Reinigung der Reaktionslösung muss das Lauroylperoxid durch Abkühlen der Lösung auf 0°C auskristallisiert werden.

GB 1 135 372 beschreibt ein Verfahren zur Herstellung von Lauroylperoxid unter Verwendung von Laurinsäurechlorid als Ausgangsprodukt. Die Reaktionsführung sieht zur Aufarbeitung des Produkts die Zugabe von warmem Wasser mit einer Temperatur von 45°C und eine abschließende Trocknung bei 45°C-50°C vor.

GB 950 978 beschreibt die Herstellung von Lauroylperoxid ausgehend von Laurinsäurechlorid bei Reaktionstemperaturen von 50°C bis 60°C, sodass Lauroylperoxid in Form einer Schmelze vorliegt.

Lauroylperoxid ist kommerziell in Form von Schuppen, Pasten oder als wässrige Suspension erhältlich. Für bestimmte Anwendungen ist es jedoch nötig, Lauroylperoxid in Form eines feinen Pulvers bereitzustellen. So ermöglicht ein Pulver mit niedriger Partikelgröße beispielsweise eine besonders gute Lösbarkeit in Lösungsmitteln, Harzen oder Monomeren. Um Lauroylperoxid-Rohprodukt, welches gemäß aus dem Stand der Technik bekannten Herstellungsverfahren erhalten wurde, in ein Pulver zu verarbeiten, wäre generell mindestens ein zusätzlicher Verfahrensschritt unter Vermahlung des Rohprodukts nötig. Gegebenenfalls müsste diesem Schritt auch ein weiterer Verfahrensschritt vorangehen, in dem synthetisiertes Lauroylperoxid zunächst aufgeschmolzen, gereinigt und anschließend wieder erstarrt wird. Da eine Erwärmung von Lauroylperoxid über seinen Schmelzpunkt, wie oben beschrieben, jedoch ein gewisses Sicherheitsrisiko bedingt, stellt dies weitere Anforderungen an die apparative Ausstattung und die Verfahrensdurchführung, was wiederum mit zusätzlichen Kosten verbunden ist. In diesem Zusammenhang wäre es wünschenswert, derartige Verfahrensschritte des Aufschmelzens und des Vermahlens eines Lauroylperoxid-Rohprodukts obsolet zu machen.

Demzufolge war es die Aufgabe der vorliegenden Erfindung, ein Herstellungsverfahren für Lauroylperoxid bereitzustellen, durch welches Lauroylperoxid direkt, d.h. ohne zusätzliche Verfahrensschritte zum Aufschmelzen und Vermahlen des Rohprodukts, als ein sehr feines, homogenes Pulver erhalten wird. Weiterhin sollte ein solches Verfahren einfach durchführbar und sicher sein.

Im Rahmen der vorliegenden Erfindung wurde festgestellt, dass eine solche Verfahrensführung möglich ist, wenn bei der Herstellung einem Gemisch aus Laurinsäurechlorid, Wasserstoffperoxid und anorganischer Base ein Alkan zugesetzt wird. Überraschenderweise zeichnet sich das so erhaltene Lauroylperoxidpulver zudem durch eine sehr hohe Reinheit aus.

Ein Aspekt der vorliegenden Erfindung betrifft deshalb ein Verfahren zur Herstellung von pulverförmigem Lauroylperoxid mit einer Partikelgröße gemäß d₉₀-Wert von 50 µm bis 400 µm, welches dadurch gekennzeichnet ist, dass ein Reaktionsgemisch verwendet wird, das Wasser, Laurinsäurechlorid, Wasserstoffperoxid, anorganische Base und ein Alkan umfasst, und das Verfahren bei einer vorbestimmten Temperatur im Bereich von etwa 10°C bis etwa 30°C durchgeführt wird, wobei das Lauroyl-Peroxid während des Verfahrens nicht über seinen Schmelzpunkt erwärmt wird.

Bevorzugt wird die erfindungsgemäße Herstellung von Lauroylperoxid in der wässrigen Phase durchgeführt.

Das Verfahren wird bevorzugt bei einer Temperatur im Bereich von etwa 15°C bis etwa 20 °C, durchgeführt.

Das eingesetzte Alkan kann ausgewählt werden aus der Gruppe, bestehend aus geradkettigen, verzweigtkettigen und zyklischen Alkanen und Mischungen davon. Bevorzugt sind hierbei C₅₋₁₂-Alkane, wie Pentane, Hexane, Heptane, Octane, Nonane, Decane, Undecane oder Dodecane, sowie deren Isomere, oder Mischungen davon, wie beispielsweise Petrolether. Besonders bevorzugt wird Hexan, insbesondere Isohexan verwendet.

Die anorganische Base wird bevorzugt als wässrige Lösung einer anorganischen Base eingesetzt. Als wässrige Lösung einer anorganischen

Base kann beispielsweise eine Lauge wie Natron- oder Kalilauge verwendet werden. Beispielsweise können wässrige Lösungen von Natriumhydroxid, Kaliumhydroxid, Natriumcarbonat, Kaliumcarbonat oder Calciumhydroxid und/oder Mischungen davon verwendet werden. Bevorzugt werden wässrige Lösungen von Natriumhydroxid, Kaliumhydroxid oder Mischungen davon verwendet.

Als wässrige Lösung einer anorganischen Base oder der zuvor genannten Mischungen davon kann beispielsweise eine wässrige Lösung verwendet werden, die zwischen 1 Gew.-% und 50 Gew.-% anorganische Base umfasst. Es kann beispielsweise eine wässrige Lösung verwendet werden, die 10 Gew.-%, 15 Gew.-%, 20 Gew.-%, 25 Gew.-%, 30 Gew.-%, 40 Gew.-% oder 50 Gew.-% anorganische Base umfasst. Bevorzugt wird 25%-ige Natron- oder Kalilauge verwendet, d.h. eine wässrige Lösung, welche 25 Gew.-% Natrium- oder Kaliumhydroxid umfasst.

Der pH-Wert des Reaktionsgemischs liegt im Bereich von pH 9 bis pH 14, bevorzugt etwa pH 11 bis etwa pH 14, stärker bevorzugt etwa pH 12 bis etwa pH 14, nochmals stärker bevorzugt etwa pH 13 bis etwa pH 14, am stärksten bevorzugt beträgt er etwa pH 14.

Wasserstoffperoxid wird bevorzugt in Form einer wässrigen Lösung verwendet. Hierbei kann eine Lösung beliebiger Konzentration eingesetzt werden. Die wässrige Wasserstoffperoxidlösung kann beispielsweise etwa 5 Gew.-%, etwa 10 Gew.-%, etwa 20 Gew.-%, etwa 30 Gew.-%, etwa 40 Gew.-%, etwa 50 Gew.-%, etwa 60 Gew.-%, etwa 70 Gew.-%, oder etwa 80 Gew.-% Wasserstoffperoxid umfassen.

Der molare Überschuss an Wasserstoffperoxid über die theoretisch notwendige Menge im Reaktionsgemisch sollte etwa 30% bis etwa 80% betragen, bevorzugt etwa 50% bis etwa 60%. Der Begriff "theoretisch notwendige Menge" für Wasserstoffperoxid bezeichnet die Menge Wasserstoffperoxid, die eingesetzt werden müsste, um im Reaktionsgemisch ein Stoffmengen-Äquivalent Wasserstoffperoxid pro zwei Stoffmengen-Äquivalente Laurinsäurechlorid bereitzustellen. Für das Verhältnis von eingesetztem Wasserstoffperoxid zu eingesetztem Laurinsäurechlorid hat es sich jedoch als vorteilhaft erwiesen, Wasserstoffperoxid gegenüber der theoretisch notwendigen Menge in dem oben definierten Überschuss einzusetzen, um eine bessere Ausbeute an Lauroylperoxid zu erhalten.

Die Wasserstoffperoxidkonzentration im Reaktionsgemisch zu Beginn der Reaktion kann von 0,5 Gew.-% bis 10 Gew.-%, bevorzugt von 1 Gew.-% bis 5 Gew.-%, besonders bevorzugt von 2 Gew.-% bis 3 Gew.-% betragen.

Im Reaktionsgemisch sollte mindestens je ein Stoffmengen-Äquivalent anorganische Base pro Stoffmengen-Äquivalent Laurinsäurechlorid eingesetzt werden. Es ist jedoch bevorzugt, demgegenüber einen gewissen Überschuss an anorganischer Base einzusetzen, wobei der molare Überschuss über die theoretisch notwendige Menge im Reaktionsgemisch so hoch sein sollte wie für Wasserstoffperoxid.

Das Masseverhältnis von Alkan zu Laurinsäurechlorid im Reaktionsgemisch kann im Bereich von etwa 1:10 bis etwa 1:1 liegen, bevorzugt etwa 1:5 bis etwa 1:1, stärker bevorzugt etwa 1:4,5 bis etwa 1:2, noch stärker bevorzugt beträgt es ungefähr 1:4. Prinzipiell kann in diesem Masseverhältnis aber auch Alkan im Überschuss eingesetzt werden.

Das Masseverhältnis von wässriger Phase zu Alkan im Reaktionsgemisch kann im Bereich von etwa 100:10 bis etwa 100:1 liegen, bevorzugt im Bereich von 100:8 bis 100:2, besonders bevorzugt beträgt es ungefähr 100:4, wobei die wässrige Phase Wasser, Wasserstoffperoxid und anorganische Base umfasst.

Das Reaktionsgemisch kann weitere Komponenten enthalten. Diese weiteren Komponenten können beispielsweise ausgewählt sein aus der Gruppe, bestehend aus Stabilisatoren für Wasserstoffperoxid, Tensiden, Alkoholen und Kombinationen davon. Als Stabilisatoren für Wasserstoffperoxid können Komplexbildner verwendet werden, bevorzugt wird Ethylendiamintetraessigsäure und/oder deren Salze verwendet.

Die erfindungsgemäße Verfahrensführung ermöglicht den folgenden Synthesemechanismus. Wasserstoffperoxid und Natronlauge reagieren in wässriger Phase zu Natriumperoxid. Das zugegebene Alkan ist nicht mit Wasser mischbar und stellt eine organische Phase im Reaktionsgemisch bereit, welche bevorzugt im Vergleich zur wässrigen Phase einen deutlich kleineren Anteil am Reaktionsgemisch ausmacht. Das weiterhin zugegebene Laurinsäurechlorid löst sich zunächst in dieser organischen Phase. Anschließend reagiert es an der Grenzfläche zur wässrigen Phase analog der Schotten-Baumann-Reaktion schnell mit Natriumperoxid zu Lauroylperoxid. Dieses ist bei den gemäß vorliegender Erfindung verwendeten Synthesetemperaturen im Alkan generell nicht löslich und kristallisiert, wodurch eine Suspension von Lauroylperoxid in wässriger Phase erhalten wird. Im Gegensatz zu bisher bekannten Synthesereaktionen wird direkt pulverförmiges Lauroylperoxid erhalten, welches sich darüberhinaus durch die hierin beschriebenen vorteilhaften Eigenschaften auszeichnet. Selbstverständlich ist das erfindungsgemäße Verfahren nicht zwingend auf den beschriebenen Synthesemechanismus beschränkt.

Das erfindungsgemäße Verfahren kann als Chargen- /Batchverfahren oder als kontinuierliches Verfahren durchgeführt werden, bevorzugt ist ein Chargen-/Batchverfahren.

Das erfindungsgemäße Verfahren kann in jedem geeigneten Reaktor durchgeführt werden. Bevorzugt wird bei der Durchführung des erfindungsgemäßen Verfahrens ein Rührreaktor verwendet.

Das erfindungsgemäße Verfahren umfasst bevorzugt die folgenden Schritte:
i) Bereitstellen einer wässrigen Lösung, umfassend Wasserstoffperoxid und eine anorganische Base;
ii) Zugeben von Alkan;
iii) Temperaturkontrolliertes Zugeben von Laurinsäurechlorid unter Rühren;
iv) Temperaturkontrolliertes Fortsetzen des Rührens;
v) Filtrieren des entstandenen Niederschlags, bevorzugt mittels einer Nutsche;
vi) Waschen des Niederschlags, bevorzugt mit Wasser;
vii) gegebenenfalls Trocknen des Niederschlags.

Unter einem temperaturkontrollierten Schritt ist im Sinne dieser Erfindung ein Verfahrensschritt unter Einhaltung einer vorbestimmten Temperatur im Bereich von etwa 10°C bis etwa 30°C, bevorzugt im Bereich von etwa 15°C bis etwa 20 °C zu verstehen.

Erfindungsgemäß kann eine wässrige Lösung, umfassend Wasserstoffperoxid, anorganische Base und Alkan, vorgelegt und auf eine vorbestimmte Temperatur im Bereich von etwa 10°C bis etwa 30°C, bevorzugt im Bereich von etwa 15°C bis etwa 20 °C gekühlt werden. Die Kühlung erfolgt beispielsweise mittels einer Mantel- oder Schlangenkühlung oder im Fall eines Rohrreaktors mittels eines Wärmetauschers. Anschließend kann ein temperaturkontrolliertes Zugeben von Laurinsäurechlorid erfolgen. Das Rühren des Reaktionsgemischs erfolgt beispielsweise mittels eines Propellerrührers mit Rührgeschwindigkeiten im Bereich von etwa 1000 bis etwa 3000 U/min.

Anstelle einer sequentiellen Zugabe von Alkan und Laurinsäurechlorid in zwei separaten Schritten ii) und iii) können das Alkan oder zumindest eine Teilmenge des Alkans und das Laurinsäurechlorid erfindungsgemäß auch vorab miteinander gemischt und anschließend zu der wässrigen Lösung bzw. zu der wässrigen Lösung, welche bereits eine Teilmenge des Alkans umfasst, zugegeben werden. Dementsprechend können in obigem Schema die Schritte ii) und iii) auch teilweise oder vollständig zusammengefasst werden.

Ein temperaturkontrolliertes Zugeben von Laurinsäurechlorid bzw. von einem Gemisch, welches Alkan und Laurinsäurechlorid umfasst, erfolgt bevorzugt durch Zutropfen. Bevorzugt erfolgt diese Zugabe während eines Zeitraums von ungefähr 10 Minuten bis 30 Minuten unter Rühren. Nach vollständiger Zugabe des Laurinsäurechlorids wird das Rühren fortgesetzt, bevorzugt für einen Zeitraum von ungefähr 10 Minuten bis ungefähr 30 Minuten.

Bei der Zugabe von Laurinsäurechlorid bzw. von einem Gemisch, welches Alkan und Laurinsäurechlorid umfasst, wird die Kühlung so kontrolliert, dass die Temperatur des Reaktionsgemischs den oben definierten gewünschten Temperaturbereich nicht überschreitet. Es ist bevorzugt, dass zumindest während der Schritte ii) bis iv) durch Kühlung der gewünschte Temperaturbereich eingehalten wird. Wahlweise können zusätzlich auch einer oder mehrerer oder alle der Schritte i) und v) bis vii) bei der selben Temperatur erfolgen.

Bei dem nach Schritt iv) erhaltenen Niederschlag handelt es sich um pulverförmiges Lauroylperoxid, welches ohne vorherige Trennung der organischen und wässrigen Phase aufgearbeitet werden kann. Eine Abtrennung des Lauroylperoxidniederschlags vom Reaktionsgemisch kann durch beliebige Verfahren, beispielsweise unter Verwendung von Filtern, Nutschen, Bandfiltern oder Zentrifugen erreicht werden. Bei Verwendung einer Nutsche kann der Filterkuchen beispielsweise direkt auf der Nutsche mit Wasser gewaschen werden oder nochmals separat mit Wasser aufgerührt und wieder abfiltriert werden. Ein auf diese Weise erhaltenes noch wasserfeuchtes Lauroylperoxidpulver kann einen Gehalt von ungefähr 75 Gew.-% aufweisen und anschließend mittels Luft oder Vakuum getrocknet werden. Das erhaltene pulverförmige Lauroylperoxid liegt in sehr feiner und homogener Form vor und weist eine hohe Reinheit auf.

In aus dem Stand der Technik bekannten Verfahren zur Herstellung von Lauroylperoxid ist oft ein Aufschmelzen des Lauroylperoxid-Rohprodukts nötig, beispielsweise zu Zwecken der Aufreinigung. Im Gegensatz hierzu ist es erfindungswesentlich, dass weder das Reaktionsgemisch, noch das erhaltene Lauroylperoxidpulver zu irgendeinem Zeitpunkt des Verfahrens auf eine Temperatur erwärmt werden, die über dem Schmelzpunkt von Lauroylperoxid liegt. Hierdurch wird zum einen eine höhere Sicherheit des Verfahrens gewährleistet, zum anderen wird eine einfachere und kostengünstigere Durchführung ermöglicht.

Ein weiterer Aspekt der vorliegenden Erfindung betrifft ein durch das erfindungsgemäße Verfahren hergestelltes pulverförmiges Lauroylperoxid, mit einer Partikelgröße gemäß d₉₀-Wert von 50 µm bis 400 µm. Wie im Folgenden erläutert wird, zeichnet sich dieses Produkt dadurch aus, dass es eine sehr feine und homogene Pulverform und darüberhinaus eine sehr hohe Reinheit ausweist. Lauroylperoxid wird in dem erfindungsgemäßen Verfahren in dieser Form erhalten, ohne dass weitere Verfahrensschritte wie beispielsweise Vermahlen nötig sind.

Das gemäß dem erfindungsgemäßen Verfahren hergestellte Lauroylperoxid weist einen d₉₀-Wert im Bereich von 50 µm bis 400 µm, und einen d₅₀-Wert im Bereich von 10 µm bis 300 µm, bevorzugt im Bereich von 10 µm bis 250 µm auf. d₅₀- und d₉₀-Wert sind so definiert, dass 50 % bzw. 90 % der in einer Stichprobe enthaltenen Partikel einen geringeren Partikeldurchmesser als den jeweiligen d₅₀- bzw. d₉₀-Wert der Probe aufweisen. Für das gemäß dem erfindungsgemäßen Verfahren hergestellte Lauroylperoxid weist das Verteilungsspektrum der Partikelgröße eine einzelne, sehr homogene Bande auf.

Das gemäß dem erfindungsgemäßen Verfahren hergestellte pulverförmige Lauroylperoxid zeichnet sich bereits direkt nach dem Waschen mit Wasser in Schritt vi) durch eine sehr hohe Reinheit aus. So weist es einen Trockengehalt von mehr als 98,5 Gew.-%, bevorzugt mehr als 99,0 Gew.-%, stärker bevorzugt mehr als 99,2 Gew.-%, noch stärker bevorzugt mehr als 99,5 Gew.-% Lauroylperoxid auf. Der Trockengehalt an Laurinpersäure beträgt weniger als 0,1 Gew.-%, bevorzugt sogar weniger als 0,05 Gew.-%, stärker bevorzugt weniger als 0,02 Gew.-%. Der Rest-Chlor-Gehalt beträgt weniger als 200 ppm, bevorzugt weniger als 100 ppm, stärker bevorzugt weniger als 70 ppm.

Das gemäß dem erfindungsgemäßen Verfahren hergestellte pulverförmige Lauroylperoxid wird in einer Ausbeute von gleich oder mehr als 90%, bevorzugt gleich oder mehr als 95 %, stärker bevorzugt gleich oder mehr als 96 %, noch stärker bevorzugt gleich oder mehr als 98 % erhalten. Die Ausbeute bezieht sich auf die erhaltenen Stoffmengen an Lauroylperoxid bezogen auf die Stoffmengen an eingesetztem Laurinsäurechlorid, wobei theoretisch aus zwei Äquivalenten Laurinsäurechlorid ein Äquivalent Lauroylperoxid entsteht.

### Figuren

- Figur 1:: Partikelgrößenverteilung von erfindungsgemäß hergestelltem Lauroylperoxid
- Figur 2:: Partikelgrößenverteilung von Lauroylperoxid hergestellt gemäß Stand der Technik

### Beispiele

### Beispiel 1: Herstellung von pulverförmigem Lauroylperoxid

1250 g Wasser, 42 g (0,86 mol) Wasserstoffperoxid 70%, 252 g (1,58 mol) Natronlauge 25% und 60 g Isohexan werden vorgelegt und auf 16 °C gekühlt. 240 g (1,10 mol) Laurinsäurechlorid werden binnen 20 Minuten unter Kühlen bei 16 bis 18 °C zugetropft. Nach der Zugabe wird die Reaktionsmischung 20 Minuten bei der gleichen Temperatur weitergerührt. Es wird über eine Nutsche filtriert und der erhaltene Feststoff mehrfach mit insgesamt 4 I Wasser gewaschen. Man erhält 291 g wasserfeuchtes Produkt mit einem Gehalt von 75 %, was einer Ausbeute von 98 % entspricht. Nach dem Trocknen an der Luft oder im Vakuum lag der Lauroylperoxidgehalt bei 99,1 %. Der Chlorgehalt im Produkt beträgt 60 ppm, der Laurinpersäuregehalt 0,01%.

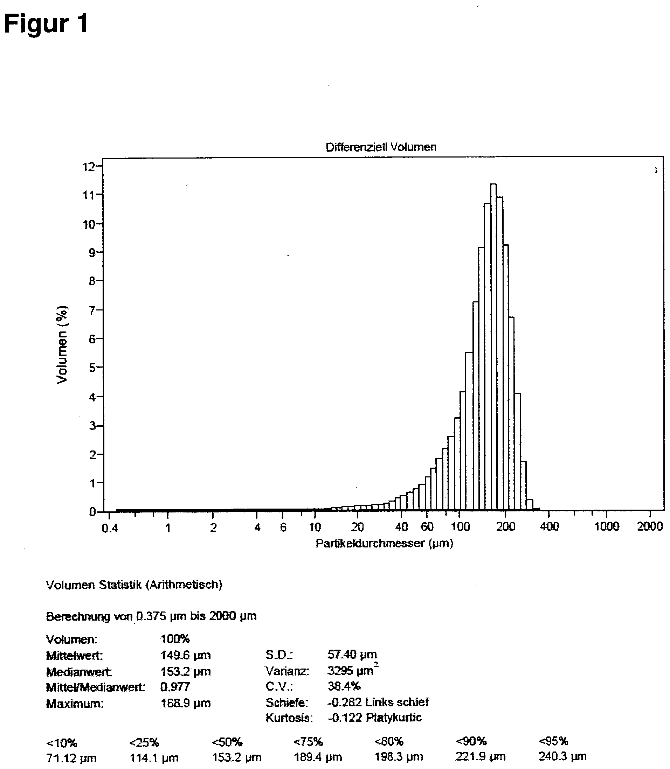

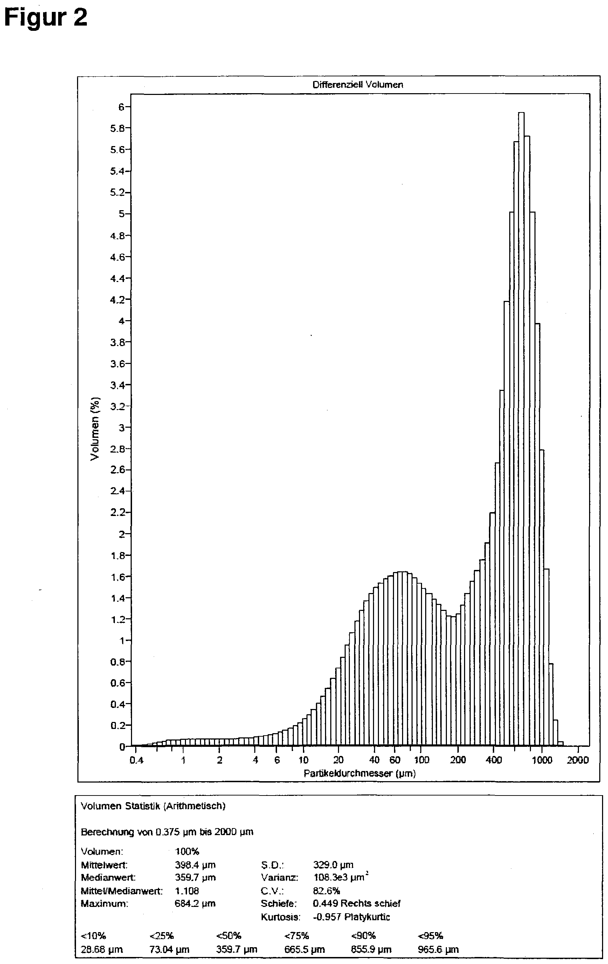

Die Partikelgrößenverteilung ist ohne weitere Vermahlung sehr homogen und weist einen d₅₀-Wert von 153 µm und einen d₉₀-Wert von 222 µm auf. Das Spektrum ist in Fig. 1 aufgeführt. Zum Vergleich wurde ein Spektrum eines am Markt erhältlichen Lauroylperoxidpulvers aufgenommen (Fig. 2). Es weist einen d₅₀-Wert von 360 µm und einen d₉₀-Wert von 856 µm auf.

### Beispiel 2: Verwendung von Lauroylperoxid in der Harzhärtung

Lauroylperoxid (LP) wird nicht nur bei der PVC-Herstellung, sondern beispielsweise auch bei der Harzhärtung verwendet. Dazu muss es zunächst im Polystyrolharz gelöst werden. Es wurden vergleichende Auflöseversuche für kommerziell erhältliche LP-Schuppen und erfindungsgemäßes LP-Pulver durchgeführt. Dabei zeigte sich, dass sich das LP-Pulver wie erwartet schneller auflöst als die Schuppen.

**Tabelle 1: Auflösegeschwindigkeit von LP in 20 g Styrol unter Rühren**

| | Menge in g | Auflösezeit in min |
|---|---|---|
| LP-Schuppen° | 9,5 | 8 |
| LP trocken | 9,5 | 6 |
| LP-75-W* | 11,0 | 6 |

| | | |
|---|---|---|
| * wasserfeuchtes Pulver mit 75% LP-Gehalt ° nicht erfindungsgemäß | | |

Bei einem Härtungsversuch wurde die Gelzeit t_{gel}, die dabei maximal erreichte Temperatur Tₘₐₓ und die Zeit tₘₐₓ bis zum Erreichen dieser maximalen Temperatur bestimmt:
Härtung: nach DIN 16945 im Reagenzglas bei 80°C
Harz: Palatal P4
Füllmittel: Aluminiumtrihydroxid (40 Teile auf 100 Teile Harz)

| | Peroxid menge | t_{gel} in min | tₘₐₓ in min | Tₘₐₓ in °C |
|---|---|---|---|---|
| LP-Schuppen° | 1,0% | 27,9 | 31,6 | 155 |
| LP trocken | 1,0% | 24,9 | 27,8 | 163 |
| LP-75-W | 1,3% | 25,5 | 28,2 | 165 |

| | | | | |
|---|---|---|---|---|
| ° nicht erfindungsgemäß | | | | |

Man erkennt, dass die Zeiten t_{gel} und tₘₐₓ kürzer sind und Tₘₐₓ höher ist. Das LP-Pulver ist demnach aktiver als die LP-Schuppen.

## Patentansprüche

1. Verfahren zur Herstellung von pulverförmigem Lauroylperoxid mit einer Partikelgröße gemäß d₉₀-Wert von 50 µm bis 400 µm, **dadurch gekennzeichnet, dass** ein Reaktionsgemisch verwendet wird, das Wasser, Laurinsäurechlorid, Wasserstoffperoxid, anorganische Base und ein Alkan umfasst und das Verfahren bei einer vorbestimmten Temperatur im Bereich von etwa 10 °C bis etwa 30 °C durchgeführt wird, wobei das Lauroylperoxid während des Verfahrens nicht über seinen Schmelzpunkt erwärmt wird.

2. Verfahren nach Anspruch 1, **dadurch gekennzeichnet, dass** die Herstellung von Lauroylperoxid in der wässrigen Phase durchgeführt wird.

3. Verfahren nach einem der vorhergehenden Ansprüche, **dadurch gekennzeichnet, dass** es bei einer vorbestimmten Temperatur im Bereich von etwa 15 °C bis etwa 20 °C, durchgeführt wird.

4. Verfahren nach einem der vorhergehenden Ansprüche, **dadurch gekennzeichnet, dass** das Alkan ausgewählt wird aus der Gruppe, bestehend aus geradkettigen, verzweigtkettigen und zyklischen Alkanen und Mischungen davon, insbesondere C₅₋₁₂-Alkanen, bevorzugt Isohexan.

5. Verfahren nach einem der vorhergehenden Ansprüche, **dadurch gekennzeichnet, dass** als anorganische Base eine Lauge, bevorzugt eine wässrige Lösung von Natriumhydroxid, Kaliumhydroxid oder Mischungen davon, verwendet wird.

6. Verfahren nach einem der vorhergehenden Ansprüche, **dadurch gekennzeichnet, dass** der molare Überschuss an Wasserstoffperoxid über die theoretisch notwendige Menge im Reaktionsgemisch etwa 30 % bis etwa 80 % beträgt, bevorzugt etwa 50 % bis etwa 60 %.

7. Verfahren nach einem der vorhergehenden Ansprüche, **dadurch gekennzeichnet, dass** das Masseverhältnis von Alkan zu Laurinsäurechlorid im Reaktionsgemisch im Bereich von etwa 1:10 bis etwa 1:1 liegt, bevorzugt ungefähr 1:4 beträgt.

8. Verfahren nach einem der vorhergehenden Ansprüche, **dadurch gekennzeichnet, dass** das Reaktionsgemisch weitere Komponenten, ausgewählt aus der Gruppe, bestehend aus Stabilisatoren für Wasserstoffperoxid, Tensiden, Alkoholen und Kombinationen davon, umfasst.

9. Verfahren nach Anspruch 8, **dadurch gekennzeichnet, dass** als Stabilisatoren für Wasserstoffperoxid Komplexbildner, bevorzugt Ethylendiamintetraessigsäure und/oder deren Salze verwendet werden.

10. Verfahren nach einem der vorhergehenden Ansprüche, **dadurch gekennzeichnet, dass** die Reaktion in einem Rührreaktor und/oder als Chargenverfahren durchgeführt wird.

11. Verfahren nach einem der vorhergehenden Ansprüche, umfassend die Schritte:
i) Bereitstellen einer wässrigen Lösung, umfassend Wasserstoffperoxid und eine anorganische Base;
ii) Zugeben von Alkan;
iii) Temperaturkontrolliertes Zugeben von Laurinsäurechlorid unter Rühren;
iv) Temperaturkontrolliertes Fortsetzen des Rührens;
v) Filtrieren des entstandenen Niederschlags, bevorzugt mittels einer Nutsche;
vi) Waschen des Niederschlags, bevorzugt mit Wasser;
vii) gegebenenfalls Trocknen des Niederschlags.

12. Verfahren nach Anspruch 11, **dadurch gekennzeichnet, dass** zumindest während der Schritte ii) bis iv) durch Kühlung eine vorbestimmte Temperatur im Bereich von etwa 10 °C bis weniger als etwa 30 °C eingehalten wird.

13. Verfahren nach Anspruch 11 oder Anspruch 12, **dadurch gekennzeichnet, dass** zumindest während der Schritte ii) bis iv) durch Kühlung eine vorbestimmte Temperatur im Bereich von etwa 15 °C bis etwa 20 °C, eingehalten wird.

14. Pulverförmiges Lauroylperoxid, erhältlich durch das Verfahren nach einem der vorhergehenden Ansprüche, wobei das Lauroylperoxid einen d₉₀-Wert im Bereich von 50 µm bis 400 µm aufweist.

15. Pulverförmiges Lauroylperoxid nach Anspruch 14, wobei das Lauroylperoxid einen d₅₀-Wert im Bereich von 10 µm bis 300 µm, bevorzugt 10 µm bis 250 µm, aufweist.

## Claims

1. Method for producing powdered lauroyl peroxide having a particle size according to a d₉₀ value of from 50 µm to 400 µm, **characterised in that** a reaction mixture is used which comprises water, lauric acid chloride, hydrogen peroxide, an inorganic base and an alkane, and the method is carried out at a predetermined temperature in the range of from approx. 10°C to approx. 30°C, the lauroyl peroxide not being heated above the melting point thereof during the method.

2. Method according to claim 1, **characterised in that** the production of lauroyl peroxide is carried out in the aqueous phase.

3. Method according to either of the preceding claims, **characterised in that** it is carried out at a predetermined temperature in the range of from approx. 15°C to approx. 20°C.

4. Method according to any of the preceding claims, **characterised in that** the alkane is selected from the group consisting of straight-chain, branched-chain and cyclic alkanes and mixtures thereof, in particular C₅₋₁₂ alkanes, preferably isohexane.

5. Method according to any of the preceding claims, **characterised in that** a lye, preferably an aqueous solution of sodium hydroxide, potassium hydroxide or mixtures thereof, is used as an inorganic base.

6. Method according to any of the preceding claims, **characterised in that** the molar excess of hydrogen peroxide over the theoretically required amount in the reaction mixture is approx. 30% to approx. 80%, preferably approx. 50% to approx. 60%.

7. Method according to any of the preceding claims, **characterised in that** the mass ratio of alkane to lauric acid chloride in the reaction mixture is in the range of from approx. 1:10 to approx. 1:1, preferably around 1:4.

8. Method according to any of the preceding claims, **characterised in that** the reaction mixture comprises further components selected from the group consisting of stabilisers for hydrogen peroxide, surfactants, alcohols and combinations thereof.

9. Method according to claim 8, **characterised in that** complexing agents, preferably ethylenediaminetetraacetic acid and/or the salts thereof are used as stabilisers for hydrogen peroxide.

10. Method according to any of the preceding claims, **characterised in that** the reaction is carried out in a stirred-tank reactor and/or as a batch process.

11. Method according to any of the preceding claims, comprising the following steps:
i) providing an aqueous solution comprising hydrogen peroxide and an inorganic base;
ii) adding an alkane;
iii) adding lauric acid chloride in a temperature-controlled manner while stirring;
iv) continuing the stirring in a temperature-controlled manner;
v) filtering the resulting precipitate, preferably by means of a suction filter;
vi) washing the precipitate, preferably with water; and
vii) drying the precipitate, if necessary.

12. Method according to claim 11, **characterised in that** at least during steps ii) to iv) a predetermined temperature in the range of from approx. 10°C to less than approx. 30°C is maintained by cooling.

13. Method according to either claim 11 or claim 12, **characterised in that** at least during steps ii) to iv) a predetermined temperature in the range of from approx. 15°C to less than approx. 20°C is maintained by cooling.

14. Powdered lauroyl peroxide, obtainable by the method according to any of the preceding claims, wherein the lauroyl peroxid has a d₉₀ value in the range of from 50 µm to 400 µm.

15. Powdered lauroyl peroxide according to claim 14, wherein the lauroyl peroxide has a d₅₀ value in the range of from 10 µm to 300 µm, preferably 10 µm to 250 µm.

## Revendications

1. Procédé de production de peroxyde de lauryle en poudre comportant une taille de particule selon la valeur d₉₀ de 50 µm à 400 µm, **caractérisé en ce qu'**un mélange réactionnel est utilisé, qui comprend de l'eau, du chlorure d'acide laurique, du peroxyde d'hydrogène, une base inorganique et un alcane et **en ce que** le procédé est mis en oeuvre à une température prédéterminée dans une plage d'environ 10 °C à environ 30 °C, dans lequel le peroxyde de lauryle n'est pas chauffé au-dessus de son point de fusion pendant le procédé.

2. Procédé selon la revendication 1, **caractérisé en ce que** la production de peroxyde de lauryle est réalisée en phase aqueuse.

3. Procédé selon l'une quelconque des revendications précédentes, **caractérisé en ce qu'**il est mis en oeuvre à une température prédéterminée dans une plage d'environ 15 °C à environ 20 °C.

4. Procédé selon l'une quelconque des revendications précédentes, **caractérisé en ce que** l'alcane est choisi dans le groupe constitué par des alcanes à chaîne linéaire, alcanes à chaîne ramifiée et alcanes cycliques et des mélanges de ceux-ci, en particulier des alcanes en C₅ à C₁₂, de préférence de l'isohexane.

5. Procédé selon l'une quelconque des revendications précédentes, **caractérisé en ce que** l'on utilise comme base inorganique une solution alcaline, de préférence une solution aqueuse d'hydroxyde de sodium, d'hydroxyde de potassium ou de mélanges de ceux-ci.

6. Procédé selon l'une quelconque des revendications précédentes, **caractérisé en ce que** l'excédent molaire de peroxyde d'hydrogène par rapport à la quantité théoriquement nécessaire dans le mélange réactionnel est d'environ 30 % à environ 80 %, de préférence d'environ 50 % à environ 60 %.

7. Procédé selon l'une quelconque des revendications précédentes, **caractérisé en ce que** le rapport en poids d'alcane au chlorure d'acide laurique dans le mélange réactionnel se situe dans une plage d'environ 1:10 à environ 1:1, de préférence est d'approximativement 1:4.

8. Procédé selon l'une quelconque des revendications précédentes, **caractérisé en ce que** le mélange réactionnel comprend d'autres composants choisis dans le groupe constitué par des stabilisants du peroxyde d'hydrogène, des tensioactifs, des alcools et des combinaisons de ceux-ci.

9. Procédé selon la revendication 8, **caractérisé en ce que** l'on utilise comme stabilisants du peroxyde d'hydrogène, des agents complexants, de préférence de l'acide éthylènediamine-tétra-acétique et/ou ses sels.

10. Procédé selon l'une quelconque des revendications précédentes, **caractérisé en ce que** la réaction est mise en oeuvre dans un réacteur agité et/ou par un procédé discontinu.

11. Procédé selon l'une quelconque des revendications précédentes, comprenant les étapes de :
i) mise à disposition d'une solution aqueuse, comprenant du peroxyde d'hydrogène et une base inorganique :
ii) addition d'alcane ;
iii) addition à température contrôlée de chlorure d'acide laurique sous agitation ;
iv) poursuite de l'agitation à température contrôlée ;
v) filtrage du précipité obtenu, de préférence au moyen d'un entonnoir filtrant ;
vi) lavage du précipité, de préférence à l'eau ;
vii) éventuellement séchage du précipité.

12. Procédé selon la revendication 11, **caractérisé en ce qu'**au moins pendant les étapes ii) à iv), une température prédéterminée est maintenue par refroidissement dans une plage d'environ 10 °C à moins d'environ 30 °C.

13. Procédé selon la revendication 11 ou la revendication 12, **caractérisé en ce qu'**au moins pendant les étapes ii) à iv), par refroidissement, une température prédéterminée est maintenue dans une plage d'environ 15 °C à environ 20 °C.

14. Peroxyde de lauryle en poudre, pouvant être obtenu par le procédé selon l'une quelconque des revendications précédentes, dans lequel le peroxyde de lauryle présente une valeur d₉₀ dans la plage de 50 µm à 400 µm.

15. Peroxyde de lauryle en poudre selon la revendication 14, dans lequel le peroxyde de lauryle présente une valeur d₅₀ dans la plage de 10 µm à 300 µm, de préférence de 10 µm à 250 µm.
